# EUROPEAN PATENT SPECIFICATION

(11) **EP 1 443 083 B1**
(45) Date of publication and mention of the grant of the patent: **27.02.2008**
(21) Application number: 04000816.1
(22) Date of filing: 16.01.2004
(51) Int. Cl.: C09C 1/00, C09D 5/36, C09D 11/02, C09D 11/00, C09K 11/08

(54) **Pearlescent pigments based on selectively absorbing layers of chalcogenide, oxychalcogenide and mixed chalcogenides**
Perlglanzpigmente auf Basis von aus Chalkogeniden, Oxidchalkogeniden und gemischten Chalkogeniden bestehenden, selektiv absorbierenden Schichten
Pigments d'interférence à base de couches sélectivement absorbantes se composant de chalcogenures, de oxychalcogenures et de chalcogenures melangés

(30) Priority: 03.02.2003 EP 03002302
(43) Date of publication of application: 04.08.2004
(73) Proprietor: Merck Patent GmbH, 64293 Darmstadt (DE)
(72) Inventor: Bertaux, Stephane, Dr., 91100 Villabé (FR); Reynders, Peter, Dr., 64347 Griesheim (DE); Wichmann, Jens-Uwe, Dr., 79117 Freiburg (DE)

(56) References cited:
- EP-A- 0 579 091
- GB-A- 850 449
- GB-A- 1 116 257
- US-B1- 6 419 736

## Description

The present invention relates to novel pearlescent pigments based on substrates comprising at least one selectively light absorbing layer which consists of a chalcogenide and/or oxychalcogenide, preferably sulfides or oxysulfides excluding rare earth and yttrium sulfides and rare earth and yttrium oxysulfides. The coatings can be directly prepared by the precipitation of chalcogenides in liquid suspension onto the substrates. Preferably, metal oxides or mixed metal oxides are coated onto the substrate; the resulting precursor is transferred into a furnace and calcined under a sulfurizing gas flow to convert the oxides into oxysulfides and/or sulfides depending on the reaction parameters. The conversion to sulfides and/or oxysulfides is preferably carried out in a fluidized bed reactor. Angle-dependent optical pigments are thus produced, which are especially useful in paints, powder coatings, paper coatings, plastics, cosmetics, inks and security-enhancing features as well as in decorative applications for foods and drugs.

Absorption pigments based on chalcogenide, oxychalcogenide and mixed chalcogenides without a layered or substrate-based structure are well known. A comprehensive overview about these substances can be found in Mane, R.S. and Lokhande, C.D., "Chemical deposition method for metal chalcogenide thin films", Materials Chemistry and Physics, 65 (2000), 1-31.

First attempts to use the advantages of these substances for the production of more sophisticated substrate-based effect pigments are described in US-A 6,063,179. This patent describes goniochromatic luster pigments based on silicon dioxide platelets coated with a non-selectively absorbing film-like layer at least partially transparent to visible light, and if desired an outer layer which consists essentially of colorless or selectively absorbing metal oxide and/or comprises a phosphate, chromate and/or vanadate. The nonselectively absorbing layer can be a metal sulfide, such as iron, cobalt, nickel, chromium, molybdenum and tungsten sulfide. These layers are non-selectively absorbing light which result in primarily black or dark colored pigments.

A layer is selectively absorbing if there is a higher or lower absorption in a certain region of the electromagnetic spectrum. The result is a real color effect different from white-gray-black (Coating (2001)(4) 135; chroma according to DIN 5033 and DIN 6174).

A number of pigments based on sulfides and oxysulfides are disclosed in DE-A 19 81 03 17. Specifically platelet-shaped substrates coated with sulfides having the formula M₂S₃ and oxysulfides having the formula M₂S₃₋ₓOₓ (0.05 ≤x ≤2.5) are mentioned in which M is a rare earth element or yttrium. The substrates are chosen from natural and synthetic mica, SiO₂-, TiO₂-, Al₂O₃-flakes, glass flakes, graphite, BiOCl, kaolin, talc, vermiculite, iron oxide flakes and metal flakes. These substrates may be uncoated or coated with one or more layers of oxides under the sulfide respectively oxysulfide layer. The sulfide respectively oxysulfide layers may be doped with one or more alkali ions, such as Na or K ions. The process for making these pigments comprises coating the substrates with an oxide, an oxide hydrate or an oxalate through a precipitation process. Then the pigments are dried, calcined between 400°C and 800°C and converted into sulfides respectively oxysulfides under S, CS₂, H₂S or a compound containing S. Due to the usage of rare earth elements, these pigments and their production are high priced what limits the usability in applications.

It was therefore an object of the present invention to provide readily available pigments with a great variety of different mass-tones which combine an viewing angle dependant interference phenomenon with the absorption color, therewith extending the range of pearlescent pigments based on chalcogenides/oxychalcogenides.

The pigments according to the present invention can surprisingly fulfill the above-mentioned objectives. Therefore, the present invention describes pearlescent pigments based on substrates comprising at least one selectively light absorbing layer which consists of chalcogenide and/or oxychalcogenide excluding rare earth and yttrium sulfides and rare earth and yttrium oxysulfides according to claim 1. Preferably the chalcogenide and/or oxychalcogenide is a metal chalcogenide and/or metal oxychalcogenide with a metal being selected from group 2 and/or 4-16 of the periodic system.

The chalcogenide-, oxychalcogenide- and mixed chalcogenide-containing coatings can be prepared by the precipitation of chalcogenides onto substrates in liquid suspension onto the substrates. For example, sodium sulfide and a metal chloride are simultaneously added to a suspension of platelet-shaped substrates leading to a hydrous metal sulfide coating onto said substrates. In a similar way, ammonium sulfide, ammonium polysulfide or sodium selenide or sodium telluride can be used. The resulting coated substrate, the so-called precursor, is separated from the mother liquid, dried and preferably calcined. The selenide or telluride main components and dopants can also be precipitated via decomposition or hydrolysis of organic precursors in gas phase, aqueous phase or non-aqueous main phase reactions, e.g. using R¹-Se-R² or R¹-Te- R² as educts with R¹ respectively R² = alkyl, aryl, Me₃Si.

However, in this invention the preferred synthesis of the new pigments is performed via a two-step process including a gas phase reaction. The first step is the synthesis of a precursor based on a substrate. The second step is a conversion process, carried out in a furnace. The pearlescent pigments according to the present invention can be produced in conventional static ovens, belt kilns or rotary kilns. However, a commercially more attractive product with less agglomerates and faster reaction rates is obtained in fluidized bed reactors.

In the first step a layer of an oxide, hydroxide, mixed oxide and/or mixed hydroxide is deposited onto a substrate, thus obtaining the precursor being used in the second step. All known deposition techniques, such as aqueous precipitation processes, CVD and/or PVD processes can be used. However, preferably an aqueous precipitation process described for example in US-A 3,087,828, US-A 3,087,829, DE-A 19 59 998, DE-A 20 09 566, DE-A 22 14 545, DE-A 22 44 298, DE-A 23 13 331, DE-A 25 22 572, DE-A 31 37 808, DE-A 31 37 809, DE-A 31 51 343, DE-A 31 51 354, DE-A 31 51 355, DE-A 32 11 602, DE-A 32 35 107, WO 93/08237 and EP-A 0 763 573 is used to obtain the precursor. Halide, carbonate, oxalate, chloride or oxychloride solutions are used to precipitate oxides, hydroxides, mixed oxides and/or mixed hydroxides onto the substrates. The reaction parameters such as temperature, pH, agitation velocity and reactor geometry are optimized to yield a flat continuous layer of the insoluble oxides and/or hydroxides on the substrates. The mixed oxides and/or hydroxides are co-precipitated onto the substrates following an analogous process. Solutions of the different metal salts are mixed and then slowly added in the reactor to coat the substrate. The oxide, hydroxide, mixed oxide and/or mixed hydroxide can be doped with metal ions, silicon oxide, aluminum oxide, boron oxide, sulfur, phosphate ions and/or sulfate ions. The dopants can be used to create color effects (like rare earths, vanadium, or cobalt ions) as well as for the control of grain growth (like SiO₂ or aluminum oxide) during the subsequent second step. For the latter purpose, for example, small amounts sodium silicate or soluble borates can be added to the coating solution via the metal salt or the acidic respectively caustic solutions that are used to adjust the pH. Examples of metal ions as dopant are silicon, vanadium, chromium, aluminum, cerium, neodymium, praseodymium, selenium, cobalt, nickel and/or zinc ions, preferably vanadium and/or cobalt ions.

Substrates that can be used in the present invention as base material on which the oxides, hydroxides, mixed oxides and/or mixed hydroxides are precipitated include platelet-shaped, spherical or needle-shaped substrates. Preferably the substrates comprise but are not limited to:
Platelets: Micaceous iron oxide, natural (for example as in WO 99/48634), synthetic or doped (for example as in EP-A 0 068 311) micas (muscovite, phlogopite, fluoro-phlogopite, synthetic fluorophlogopite, talc, kaolin), basic lead carbonate, platelet-shaped barium sulfate, SiO₂-, Al₂O₃-, TiO₂-, Glass-, ZnO-, ZrO₂-, SnO₂-, BiOCl-, chromium oxide-, BN-, MgO-flakes, Si₃N₄, graphite, pearlescent pigments (including those which react under the fluidized bed conditions to nitrides, oxynitrides or by reduction to suboxides etc.) (for example EP-A 9 739 066, EP-A 0 948 571, WO 99/61529, EP-A 1 028 146, EP-A 0 763 573, US-A 5,858,078, WO 98/53012, WO 97/43348, US-A 6,165,260, DE-A 15 19 116, WO 97/46624, EP-A 0 509 352), pearlescent multilayer pigments (for example EP-A 0 948 572, EP-A 0 882 099, US-A 5,958,125, US-A 6,139,613) and/or metals. Preferably, the metal is aluminum and/or titanium, most preferably passivated by inorganic treatment.
Spheres: coated SiO₂ spheres (for example EP-A 0 803 550, EP-A 1 063 265, JP-A 11 322 324), uncoated SiO₂ spheres (Ronaspheres^{®} , all spheres described as starting materials in EP-A 0 803 550, EP-A 1 063 265, JP-A 11 322 324), micro bubbles (US-A 4,985,380), as well as needle-shaped metal oxides, preferably iron oxide.

The size of the substrates is not critical. The mean diameter of the substrates and hence the resulting pigments can vary between 1 and 500 µm, preferably 5 and 50 µm. Preferably, the mean diameter in the case of for example the platelet-shaped substrates can vary between 5 and 200 µm, preferably between 10 and 150 µm. The mean diameter of the spherical substrates can vary between 10 nm and 100 µm, preferably between 500 nm and 50 µm and most preferably from 1 to 20 µm. Such substrates are commercially available or can be obtained by known processes.

The chalcogenide and/or oxychalcogenide layer can be coated directly onto the substrate as described above. In the same way nitride respectively oxynitride layer coated substrates can be used as substrates to precipitate the optical layers as described above.

In the second step for the production of pigments according to the present invention, the oxides, hydroxides, mixed oxides and/or mixed hydroxides obtained in the above described first step are converted into chalcogenides and/or oxychalcogenides. This can be achieved by calcination of the precursor obtained in the first step in conventional static ovens, belt kilns or rotary kilns. However, a better product with less agglomerates and faster reaction rates is obtained in fluidized bed reactors. This process can be performed batchwise or continuously. The conversion is carried out with a reactive gas, which may consist of H₂S, CS₂, sulfur and/or a mixture of these. Additionally an inert gas such as Ar or N₂, preferably N₂ may be present during the conversion. The gas composition may vary from >0 to 100 vol-%, preferably from 20 to 80 vol-% of reaction gas in inert gas.

The temperature is maintained at a fluidized bed temperature of about 700-1250°C, preferably 800°C to 1100°C. The conversion between oxides, hydroxides, mixed oxides as well as mixed hydroxides and chalcogenides and/or oxychalcogenides is carried out depending on the different parameters, such as gas flow rates, reaction time or temperature profiles. The longer the reaction time the higher the chalcogenide-to-oxychalcogenide ratio. Consequently the reaction time determines obtained structure of the compound. The color and the color strength of the pearlescent pigments according to the present invention is associated to a determined structure that is why the reaction time has to be well controlled. In addition for the same reason the temperature control is necessary. The control and optimization of the process parameters can be performed b any person skilled in the art.

In order to maintain the almost ideal conditions prevalent in an homogeneous fluid bed in comitercurrent/cocurrent, contacting special devices are used. Instabilities like formation of channels or of bubbles in the bed are instantly destroyed by vibrations or agitating facilities.

If the reaction with the reaction gas is not carried out to full completeness, mixtures of phases can be obtained including gradient of phase concentration through the layer thickness. These incompletely reacted products can be advantageous with respect to a desired color shade.

The thickness of the chalcogenide and/or oxychalcogenide layers can vary between 5 and 500 nm, yielding slight shades and flat angle color effect at low thickness and very pronounced hiding at high thickness. For the optimal interference effect, the preferred thickness is 50-350 nm, especially preferred 80-200 nm.

The interference color is determined by the optical thickness, which is the geometrical thickness of the layer multiplied by the refractive index (Pfaff, G.; Reynders, P. "Angle-dependent optical effects deriving from submicron structures of films and pigments", Chemical Review, 99 (1999), p.1963-1981). The latter is a strong function of the chosen material but is in general not known for the rather new materials mentioned in this invention. The mass tone of the absorbing pigments is as well a function of the layer thickness. Therefore, the desired color effect is empirically optimized by adjustment of the amount of precursor, by this means controlling the precursor layer thickness, and regulation of the conversion reaction with the reactive gases.

The selectively light absorbing layer of chalcogenides and/or oxychalcogenides consists of:

### Sulfides:

1. Sulfides containing one metal:
   - AₓS_{y} with A = Zr, Fe, Zn, Mn, Co, Ni, Cu, In, Sn, Pb, Ag, Bi, Sb, As, Cr, Mo, W, Rb, Ti x>0, y>0
      doped with Ag, Al, Au, Cu, Eu, Sm or a mixture of these cations, such as ZnS:Ag, ZnS:Al, ZnS:Au, ZnS:Cu, ZnS:Cu or Al, ZnS:Cu or Al or Au, SrS:Eu or Sm, SrS:Ce or Sm, SrS:Cu and with X>0, y>0
   - AₓS_{y} doped with Euₘ, Trₙ and OₚX_{q}
      with A = alkaline earth metal ion,
      Tr = one or more trivalent rare earth metal ions,
      X = halide,
      0.01 <m<0.5, 0.01 <p<2, 0.01 <q<0.5 (values in atomic percent), or m, n, p, q are integers,
      such as SrS:Eu:Er:OCl,SrS:Eu_{0.1}:Er_{0.1}:D_{y0.1}:OCl
2. Sulfides containing two or more metals;
   preferably

   CulnS₂, CuBiS₂, CuFeS₂, Rb₄Ta₂S₁₁, CuPrS₂, Nd₂TeS₂

   EuCe₂S₄, CaLa₂S₄, Cu₅FeS₄, CuCr₂S₄,

   PbₓCa₁₋ₓLa₂S₄,

   such as Pb_{0.1}Ca_{0.9}La₂S₄, PbCeS₄
   - Na₄SiS₁₀, Ce₃Si₂IS₈, ZnSeS, ZnSe_{0,53}S_{0,47}, TISeS, K₄Nb₂S₁₀, K₆Nb₄S₂₅
   - Zn₃₋₃ₓIn_{2xy}Ga_{2x-2xy}S₃
      with 0.2<x<0.97, 0.1<y< 1
   - Znln₂S₄, Zn₂ln₂S₅
   - A₆Nb₄S₂₂
      with A = Rb, Cs

      CuGa₅S₈, Culn₅S₈
   - AgGaₓIn₅₋ₓS₈ with 0 ≤ x ≤ 3,
      such as AgGa₃ln₂S₈, AgGa₅S₈, Agln₅S₈, AgGa₄lnS₈
   - Rb₄Ti₃S₁₄, Cs₄Zr₃S₁₄, K₄Ti₃S₁₄, Tl₂TiS₄, Cs₂TiS₃, K₂TiS₃, Na₂ZrS₃, Ba₃Zr₂S₇, Cu₂HfS₃, Cu₄TiS₄, Ag₄Hf₃S₈, Ag₂HfS₃
   - KLnMS₄
      with Ln = rare earth or Y,
      M = Si, Ge,
      such as KCeSiS₄, KLaGeS₄
   - Ca(₁₋ₓ)Yb_{(2/3 x)}Defect_{(1/3 x})S with 0 ≤ x ≤ 1

### Oxysulfides;

preferably
- ZrOS, Rb₄Nb₂OS₁₀
   Na-Sr-Cu-M-O-S
   with M = Zn, Ga, In
   preferably Sr₂₋ₓNaₓCu₂ZnO₂S₂, Sr₂₋ₓNaₓCuGaO₃S with x>0,
   Sr₂Cu₂ZnO₂S₂, Sr₂CuGaO₃S, Sr₂CulnO₃S

### Selenides;

preferably

AₓSe_{y}

with A = Cd, Zn, Bi, Sb, Ni, TI, Pb, Cu, Mo, Sn, Co, with x>0, y>0
such as ZnSe, Bi₂Se₃, Sb₂Se₃, NiSe, TISe, PbSe, CuSe, MoSe₂. SnSe, CoSe
- CdₓPb_{y}Se with x>0, y>0
- CulnSe₂
- Cd₁₋ₓZnₓSe (0≤x≤0.9)
- Cd₁₋ₓFeₓSe (0≤x≤0.9)

### Sulfoselenides;

preferably

CdSₓSe_{y}, ZnSₓSe_{y} with x>0, y>0

The new pearlescent pigments can be used as substrate to precipitate further optical layers. If desired, the pigments according to the present invention can be further coated with one or more layers of metal oxides, metal oxide hydrates, metal fluorides and/or semitransparent metal layers on top of the selectively light absorbing layer. The selectively light absorbing layer can also be placed as an intermediate layer of metal oxide, metal oxide hydrate, metal fluoride and/or semitransparent metal stacks. The metal oxide can be selected from any metal oxide, preferably from titanium oxide, iron oxide, aluminium oxide, aluminium oxide hydrate, silicon oxide, silicon oxide hydrate, zirconium oxide, chromium oxide, zinc oxide, tin oxide, antimony oxide, indium oxide, potassium ferric ferro cyanides, most preferably from titanium oxide, iron oxide, aluminium oxide, aluminium oxide hydrate, silicon oxide, silicon oxide hydrate and/or mixtures thereof. The metal fluoride is preferably magnesium fluoride. The metal of the semitransparent metal layer can be selected from chromium, molybdenum, aluminium, silver, platinum, nickel, copper and/or gold, preferably from aluminium, silver. In particular, the metal oxide, metal oxide hydrate, metal fluoride and/or semitransparent metal layers are arranged as alternating layers of metal oxide, metal oxide hydrate, metal and/or metal fluoride with a refractive index n > 1.8 and a metal oxide, metal oxide hydrate, metal and/or metal fluoride with a refractive index n ≤ 1.8. Pigments according to this embodiment combine the colour of the complex phosphate system with an intensively lustrous appearance and may show an angle-dependent interference colour.

Preferred examples for metal oxides, metal oxide hydrates and/or metals with a refractive index n > 1.8 are titanium oxide, iron oxide, iron titanate, iron, chromium, silver and/or nickel, preferably titanium oxide, iron oxide, iron titanate.

Preferred examples for metal oxides, metal oxide hydrates, metals and/or metal fluorides with a refractive index n ≤ 1.8 are silicon oxide, silicon oxide hydrate, aluminium oxide, aluminium oxide hydrate, aluminium and/or magnesium fluoride.

Furthermore, the resulting pigments can be coated with inorganic and/or organic compounds to increase their weather stability respectively their photostability. Useful methods are for instance described in US-A 4,134,776, EP-A 0 649 886, WO 97/29059 and references cited therein.

The advantage of this invention is the combination of a great variety of mass-tones of the chalcogenides and derived compounds with an angle dependent interference color that is adjusted by the layer thickness of the chalcogenide/oxychalcogenide-containing layer. The applications of these new pigments are numerous, such as paints, powder coatings, paper coatings, plastics, cosmetics, inks and security-enhancing features as well as in decorative applications for foods and drugs due to the use of mainly nontoxic materials.

To create new color effects in all applications, the pearlescent pigments according to the present invention can be employed in admixture with filler pigments or transparent and hiding white, colored and black organic and inorganic pigments and also with conventional transparent, colored and black luster pigments based on metal oxide coated mica, TiO₂ flakes, SiO₂ flakes or Al₂O₃ flakes and coated or uncoated metal pigments, BiOCl pigments, platelet-shaped iron oxides or graphite flakes. The inventive pigments can be further coated with organic or inorganic layers to yield combination pigments.

Some layers of the pigments described in this invention have fluorescent, photoluminescent or electroluminescent properties itself, e.g. rare-earth doped ZnS layers. If such a time-delayed color effect is desired, for example in the field of security, optical, projections screen, safety or similar applications, and the inherent property of the invented pigments is not strong enough, physical mixtures of the invented pigments with conventional inorganic or organic fluorescent respectively luminescent pigments can be used.

The pigments and their production process according to the present invention is more illustratively demonstrated but not limited by means of the following examples.

### Examples:

### Comparison example 1

100 g of muscovite mica (Merck KGaA, diameter 10-50 µm) are suspended in 2 liters of fully deionised water. The suspension is heated to 75°C. 467 ml of a FeCl₃ solution is diluted to 1000 ml with water and is slowly added to the reactor. The pH of the solution is kept at pH 3.1 by addition of 15% aqueous sodium hydroxide solution. The preparation is filtered off, washed with completely deionised water, dried at 110°C for 12 hours and calcined at 800°C for 30 minutes. As a result, 70 g of Fe₂O₃ are precipitated onto 100 g of mica. This pigment is then put into a fluidized bed reactor, calcined under H₂S at 900°C during 12 hours. A yellow to golden FeS₂/mica pigment is obtained.

### Example 1:

100 g Iriodin^{®} 504 (Fe₂O₃/mica, Merck KGaA) are suspended in 2 liters of fully deionised water. The suspension is heated to 75°C. A ZrOCl₂ solution (72.3 g diluted in 600ml water) is slowly added to the reactor. The solution is kept at pH 3 by simultaneous addition of 15% aqueous sodium hydroxide solution. The preparation is filtered off, washed with completely deionised water and dried at 110°C for 12 hours. As a result, 50 g of ZrO₂ are precipitated onto 100 g of Iriodin^{®} 504. The pigment is then put into a fluidized bed reactor. The precursor is fluidized with N₂ to 750-850°C and then is treated with H₂S for 360 minutes. A pigment with a reddish golden color (mixed iron sulfide) and a yellow shade (zirconium oxysulfide) is obtained.

### Example 2:

100 g SiO₂ flakes (Merck KGaA, diameter 10-50 µm) are suspended in 2 liters of fully deionised water. The suspension is heated to 75°C. A ZrOCl₂ solution (72.3 g diluted in 600 ml water) is slowly added into the reactor. The solution is kept at pH 3 by addition of 15% aqueous sodium hydroxide solution. The preparation is filtered off, washed with completely deionised water, dried at 110°C for 12 hours. As a result, 50 g of hydrous ZrO₂ are precipitated onto 100 g of SiO₂ flakes. The pigment is then put into a fluidized bed reactor. The precursor is fluidized with N₂ to 750-850°C and then is converted with H₂S for 360 minutes. A pigment with a yellow color (zirconium oxysulfide) is obtained.

### Example 3:

50 g of ZrO₂:VCl₃(10:1wt.-ratio) are precipitated onto 100 g of SiO₂-flakes (Merck KGaA, diameter 10-50 µm) using a ZrOCl₂ solution (72.3 g of ZrOCl₂ solution containing 7.2 g of VCl₃ diluted into 600 ml of water) as described in the example 3. The dried pigment is then put into the fluidized bed, calcined at 800°C under H₂S for 360 minutes. A pigment with a blue color (vanadium-doped zirconium oxysulfide) was obtained.

### Example 4:

A metallized zirconium oxysulfide pigment is produced by thermally decomposing chromium hexacarbonyl in the presence of heated zirconium oxysulfide coated onto SiO₂ flakes as described in the example 3. This pigment is fluidized with nitrogen to achieve and maintain a non-bubbling fluidized bed and an oxygen free atmosphere. Then the reactor is heated to 400-450°C and kept under this condition throughout the following coating process. A stream of nitrogen loaded with chromium hexacarbonyl is prepared by passing nitrogen through a flask containing chromium hexacarbonyl, which is kept at 80°C, and introduced subsequently into the reactor. The vaporized compound is passed into the tube for about 90 minutes. About 5 nm of chromium is deposited on the zirconium oxysulfide pigment based onto SiO₂ flakes, forming a semitransparent layer. The organic by-product of the decomposition reaction is separated from the pigment into a scrubber.

### Example 5:

100 g of muscovite mica (Merck KGaA, diameter 10-50 µm) are suspended in 2 liters of fully deionised water. The suspension is heated to 75°C. A ZnCl₂ solution (84 g) is mixed with a CuCl₂ solution (10 g) and the mixture is slowly added to the reactor. The solution is kept at pH 3 by addition of 15% aqueous sodium hydroxide solution. The preparation is filtered off, washed with completely deionised water, dried at 110°C for 12 hours and then calcined at 850°C for 30 minutes. As a result, 50 g of ZnO:Cu are precipitated onto 100 g of mica. This pigment is then put into a fluidized bed reactor, calcined under H₂S at 1000°C during 12 hours. A blue ZnS:Cu/mica pigment is obtained.

### Example 6:

100 g of silica flakes (Merck KGaA, diameter 10-50 µm) are suspended in 2 liters of fully deionised water. The suspension is heated to 65°C. A solution of 83 g ZnSO₄ (120 g/l Zn) containing 0.01 mol% CuSO₄ is slowly added simultaneously with a solution of 40 g Na₂S (60 g/l Na₂S) into the reactor. The solution is kept at pH 3.5 by addition of dilute hydrochloric acid solution. The pH is increased to 7 and another small quantity of Na₂S is added in the reactor. The preparation is filtered off, washed with completely deionised water, dried at 130°C for 12 hours and then calcined at 900°C for 120 minutes. As a result, a white zinc sulfide containing pigment is obtained showing luminescence.

### Comparison example 2

100 g of muscovite mica flakes (Merck KGaA, diameter 10-50 µm) are suspended in 2 liters of fully deionised water. The suspension is heated to 75°C. A solution of 67.2 g SbCl₃ diluted with 200 g 32% HCI is slowly added simultaneously with a solution of 34.54 g Na₂S into the reactor. The solution is kept at pH 3.5 by addition of dilute hydrochloric acid solution. The preparation is filtered off, washed with completely deionised water, dried at 110°C for 12 hours and then calcined at 450°C for 60 minutes. As a result, an orange antimony(III) sulfide containing pigment is obtained.

### Example 7:

100 g of silica flakes (Merck KGaA, diameter 10-50 µm) are suspended in 2 liters of fully deionised water. The suspension is heated to 65°C. A solution of 67 g ZnSO₄ (120 g/l Zn) containing 0.01 mol% CuSO₄ is slowly added simultaneously with a solution of 16 g Na₂S (60 g/l Na₂S) and 26 g Na₂Se into the reactor. The solution is kept at pH 3.5 by addition of dilute hydrochloric acid solution. The pH is increased to 7 and another 0.5 g Na₂S is added in the reactor. The preparation is filtered off, washed with completely deionised water, dried at 130°C for 12 hours and then calcined at 600°C for 120 minutes. As a result, a white zinc sulfoselenide containing pigment is obtained.

## Claims

1. Pearlescent pigments based on substrates comprising at least one selectively light absorbing layer which consists of a sulfide AₓS_{y} with A = Zr, Fe, Zn, Mn, Co, Ni, Cu, In, Sn, Pb, Ag, Bi, Sb, As, Cr, Mo, W, Rb, Ti, x>0, y>0, doped with Ag, Al, Au, Cu, Eu, Sm or a mixture of these cations, or of a sulfide AₓS_{y} doped with Euₘ, Trₙ and OₚX_{q}, with A = alkaline earth metal ion, Tr = one or more trivalent rare earth metal ions, X = halide, 0.01<m<0.5, 0.01 <p<2, 0.01<q<0.5 (values in atomic percent), or with m, n, p, q being integers, or consists of a sulfide containing two or more metals, an oxysulfide excluding rare earth and yttrium oxysulfides, a selenide and/or sulfoselenide.

2. Pearlescent pigments according to claim 1, **characterized in that** the oxysulfide, selenide or sulfoselenide is a metal oxysulfide, selenide or sulfoselenide with a metal being selected from group 2 and/or 4-16 of the periodic system.

3. Pearlescent pigments according to claim 1 or 2, wherein the substrate is platelet-shaped, spherical or needle-shaped.

4. Pearlescent pigments according to claim 3, wherein the substrate is platelet-shaped and is of mica, SiO₂ aluminum oxide, glass, micaceous iron oxide, oxidized graphite, aluminum oxide-coated graphite, basic lead carbonate, barium sulfate, chromium oxide, BN, MgO, Si₃N₄, metal, pearlescent pigments or pearlescent multilayer pigments, or of coated or uncoated SiO₂-spheres or needle-shaped iron oxides.

5. Pearlescent pigments according to claim 4, **characterized in that** the metal is aluminum or titanium, passivated by inorganic treatment.

6. Pearlescent pigments according to any of claims 1 to 5, **characterized in that** the thickness of the selectively light absorbing layer is between 5 and 500 nm.

7. Pearlescent pigments according to one of claims 1 to 6, **characterized in that** the pigments are further coated on top of the selectively light absorbing layer with one or more layers of metal oxides, metal oxide hydrates, metal fluorides and/or semitransparent metal layers.

8. Pearlescent pigments according to claim 7, **characterized in that** the metal oxide is selected from TiO₂ and/or iron oxide and the metal is selected from Al, Mo and/or Cr.

9. Process for the preparation of a pigment according to claim 1 comprising precipitating a layer of an oxide, hydroxide, mixed oxide and/or mixed hydroxide onto a substrate and then converting the oxide, hydroxide, mixed oxide and/or mixed hydroxide into a sulfide according to claim 1 or into a sulfide containing two or more metals, an oxysulfide, a selenide or a sulfoselenide.

10. Process according to claim 9, **characterized in that** the conversion is carried out in a fluidized bed reactor.

11. Process according to claim 10 or 11, **characterized in that** the conversion is carried out with a reactive gas which consists of H₂S, CS₂, sulfur or a mixture thereof.

12. Process according to claim 11, **characterized in that** additionally an inert gas such as Ar or N₂ is present during the conversion.

13. Process according to claim 9, **characterized in that** the substrate is platelet-shaped and is of mica, SiO₂, aluminum oxide, glass, micaceous iron oxide, oxidized graphite, aluminum oxide-coated graphite, basic lead carbonate, barium sulfate, chromium oxide, BN, MgO, Si₃N₄, metal, pearlescent pigments or pearlescent multilayer pigments, or of coated or uncoated SiO₂-spheres or needle-shaped iron oxides.

14. Process according to claim 9, **characterized in that** the oxide, hydroxide, mixed oxide and/or mixed hydroxide is doped with metal ions, silicon oxide, aluminum oxide, boron oxide, sulfur, phosphate ions and/or sulfate ions.

15. Process according to claim 14, **characterized in that** the metals are selected from silicon, vanadium, chromium, aluminum, cerium, neodymium, praseodymium, cobalt, nickel and/or zinc.

16. Use of a pigment according to claim 1 in paints, powder coatings, paper coatings, plastics, cosmetics, inks, decorative applications for foods and drugs and security-enhancing features.

17. Use of a pigment according to claim 1 as phosphorescent, fluorescent or luminescent materials in security, optical or projection screen applications.

## Patentansprüche

1. Perlglanzpigmente basierend auf Substraten, die mindestens eine selektiv Licht absorbierende Schicht enthalten, die aus einem Sulfid AₓS_{y}, wobei A = Zr, Fe, Zn, Mn, Co, Ni, Cu, In, Sn, Pb, Ag, Bi, Sb, As, Cr, Mo, W, Rb, Ti, x>0, y>0, dotiert mit Ag, Al, Au, Cu, Eu, Sm oder einer Mischung dieser Kationen, oder aus einem Sulfid AₓS_{y}, dotiert mit Euₘ, Trₙ und OₚX_{q}, wobei A = Erdalkalimetallion , Tr = ein oder mehrere dreiwertige Seltenerdmetallionen, X = Halogenid, 0,01 <m<0,5, 0,01 <p<2, 0,01 <q<0,5 (Werte in Atomprozent) oder wobei m, n, p, q ganze Zahlen bedeuten, oder aus einem Sulfid mit zwei oder mehr Metallen, einem Oxysulfid mit Ausnahme der Seltenerd- und Yttriumoxysulfide, einem Selenid und/oder Sulfoselenid besteht.

2. Perlglanzpigmente nach Anspruch 1, **dadurch gekennzeichnet, dass** das Oxysulfid, Selenid oder Sulfoselenid ein Metalloxysulfid, -selenid oder -sulfoselenid mit einem Metall ist, das aus der Gruppe 2 und/oder 4-16 des Periodensystems ausgewählt ist.

3. Perlglanzpigmente nach Anspruch 1 oder 2, wobei das Substrat plättchen-, kugel- oder nadelförmig ist.

4. Perlglanzpigmente nach Anspruch 3, wobei das Substrat plättchenförmig ist und aus Glimmer, SiO₂, Aluminiumoxid, Glas, Eisenglimmer, oxidiertem Graphit, mit Aluminiumoxid beschichtetem Graphit, basischem Bleicarbonat, Bariumsulfat, Chromoxid, BN, MgO, Si₃N₄, Metall, Perlglanzpigmenten oder Perlglanz-Mehrschichtpigmenten, oder aus beschichteten oder unbeschichteten SiO₂-Kugeln oder nadelförmigen Eisenoxiden besteht.

5. Perlglanzpigmente nach Anspruch 4, **dadurch gekennzeichnet, dass** das Metall Aluminium oder Titan ist, das durch anorganische Behandlung passiviert ist.

6. Perlglanzpigmente nach einem beliebigen der Ansprüche 1 bis 5, **dadurch gekennzeichnet, dass** die Dicke der selektiv Licht absorbierenden Schicht zwischen 5 und 500 nm liegt.

7. Perlglanzpigmente nach einem der Ansprüche 1 bis 6, **dadurch gekennzeichnet, dass** die Pigmente auf der selektiv Licht absorbierenden Schicht weiterhin mit einer oder mehreren Schichten von Metalloxiden, Metalloxidhydraten, Metallfluoriden und/oder semitransparenten Metallschichten beschichtet sind.

8. Perlglanzpigmente nach Anspruch 7, **dadurch gekennzeichnet, dass** das Metalloxid aus TiO₂ und/oder Eisenoxid und das Metall aus Al, Mo und/oder Cr ausgewählt ist.

9. Verfahren zur Herstellung eines Pigments nach Anspruch 1, umfassend das Ausfällen einer Schicht eines Oxids, Hydroxids, Mischoxids und/oder Mischhydroxids auf ein Substrat und anschließendes Umwandeln des Oxids, Hydroxids, Mischoxids und/oder Mischhydroxids in ein Sulfid nach Anspruch 1 oder in ein Sulfid enthaltend zwei oder mehr Metalle, ein Oxysulfid, ein Selenid oder ein Sulfoselenid.

10. Verfahren nach Anspruch 9, **dadurch gekennzeichnet, dass** die Umwandlung in einem Wirbelschichtreaktor durchgeführt wird.

11. Verfahren nach Anspruch 10 oder 11, **dadurch gekennzeichnet, dass** die Umwandlung mit einem reaktiven Gas durchgeführt wird, das aus H₂S, CS₂, Schwefel oder einer Mischung hiervon besteht.

12. Verfahren nach Anspruch 11, **dadurch gekennzeichnet, dass** während der Umwandlung zusätzlich ein Inertgas wie Ar oder N₂ anwesend ist.

13. Verfahren nach Anspruch 9, **dadurch gekennzeichnet, dass** das Substrat plättchenförmig ist und aus Glimmer, SiO₂, Aluminiumoxid, Glas, Eisenglimmer, oxidiertem Graphit, mit Aluminiumoxid beschichtetem Graphit, basischem Bleicarbonat, Bariumsulfat, Chromoxid, BN, MgO, Si₃N₄, Metall, Perlglanzpigmenten oder Perlglanz-Mehrschichtpigmenten, oder aus beschichteten oder unbeschichteten SiO₂-Kugeln oder nadelförmigen Eisenoxiden besteht.

14. Verfahren nach Anspruch 9, **dadurch gekennzeichnet, dass** das Oxid, Hydroxid, Mischoxid und/oder Mischhydroxid mit Metallionen, Siliciumoxid, Aluminiumoxid, Boroxid, Schwefel, Phosphationen und/oder Sulfationen dotiert ist.

15. Verfahren nach Anspruch 14, **dadurch gekennzeichnet, dass** die Metalle aus Silicium, Vanadium, Chrom, Aluminium, Cer, Neodym, Praseodym, Cobalt, Nickel und/oder Zink ausgewählt sind.

16. Verwendung eines Pigmentes nach Anspruch 1 in Lacken, Pulverbeschichtungen, Papierbeschichtungen, Kunststoffen, Kosmetika, Farben, dekorativen Anwendungen für Lebens- und Arzneimittel und sicherheitsverbessernden Merkmalen.

17. Verwendung eines Pigmentes nach Anspruch 1 als Phosphoreszenz-, Fluoreszenz- oder Lumineszenzmaterialien in Sicherheits-, optischen oder Projektionsschirm-Anwendungen.

## Revendications

1. Pigments nacrants à base de substrats comprenant au moins une couche sélectivement photo-absorbante constituée d'un sulfure AₓS_{y}, avec A = Zr, Fe, Zn, Mn, Co, Ni, Cu, In, Sn, Pb, Ag, Bi, Sb, As, Cr, Mo, W, Rb, Ti, x>0, y>0, dopé par Ag, AI, Au, Cu, Eu, Sm ou un mélange de ces cations, ou d'un sulfure AₓS_{y} dopé par Euₘ, Trₙ et OₚX_{q}, avec A = un ion de métal alcalino-terreux, Tr = un ou plusieurs ions métalliques terreux rares et trivalents, X = halogénure, 0,01 <m<0,5, 0,01 <p<2, 0,01 <q<0,5 (valeurs en pourcentage atomique), ou avec m, n, p et q étant des nombres entiers, ou constituée d'un sulfure contenant deux métaux ou plus, un oxysulfure à l'exception des oxysulfures terreux rares et d'yttrium, un séléniure et/ou un sulfoséléniure.

2. Pigments nacrants selon la revendication 1, **caractérisés en ce que** l'oxysulfure, le séléniure ou le sulfoséléniure est un oxysulfure, un séléniure ou un sulfoséléniure de métal, le métal étant choisi parmi le groupe 2 et/ou 4-16 du Tableau Périodique.

3. Pigments nacrants selon la revendication 1 ou 2, dans lesquels le substrat est sous forme de plaquettes, sphérique ou d'aiguilles.

4. Pigments nacrants selon la revendication 3, dans lesquels le substrat est sous forme de plaquettes et est constitué de mica, de SiO₂, d'oxyde d'aluminium, de verre, d'oxyde de fer micacé, de graphite oxydé, de graphite revêtu d'oxyde d'aluminium, de carbonate de plomb basique, de sulfate de baryum, d'oxyde de chrome, de BN, de MgO, de Si₃N₄, de métal, de pigments nacrants ou de pigments multi-couches nacrants, ou de sphères de SiO₂ revêtues ou non revêtues ou d'oxydes de fer en forme d'aiguilles.

5. Pigments nacrants selon la revendication 4, **caractérisés en ce que** le métal est l'aluminium ou le titane, passivé par un traitement inorganique.

6. Pigments nacrants selon l'une quelconque des revendications 1 à 5, **caractérisés en ce que** l'épaisseur de la couche sélectivement photo-absorbante est comprise entre 5 et 500 nm.

7. Pigments nacrants selon l'une quelconque des revendications 1 à 6, **caractérisés en ce que** les pigments sont en outre revêtus sur la couche sélectivement photo-absorbante par une ou plusieurs couches d'oxydes métalliques, d'hydrates d'oxydes métalliques, de fluorures métalliques et/ou de couches métalliques semi-transparentes.

8. Pigments nacrants selon la revendication 7, **caractérisés en ce que** l'oxyde de métal est choisi parmi TiO₂ et/ou l'oxyde de fer, et le métal est choisi parmi Al, Mo et/ou Cr.

9. Procédé de préparation d'un pigment selon la revendication 1, comprenant la précipitation d'une couche d'un oxyde, hydroxyde, oxyde mixte et/ou hydroxyde mixte sur un substrat, puis la conversion de l'oxyde, hydroxyde, oxyde mixte et/ou hydroxyde mixte en un sulfure selon la revendication 1 ou en un sulfure contenant deux métaux ou plus, un oxysulfure, un séléniure ou un sulfoséléniure.

10. Procédé selon la revendication 9, **caractérisé en ce que** la conversion est effectuée dans un réacteur à lit fluidisé.

11. Procédé selon la revendication 10 ou 11, **caractérisé en ce que** la conversion est effectuée avec un gaz réactif constitué de H₂S, CS₂, de soufre ou d'un mélange de ceux-ci.

12. Procédé selon la revendication 11, **caractérisé en ce qu'**en outre un gaz inerte tel qu'Ar ou N₂ est présent pendant la conversion.

13. Procédé selon la revendication 9, **caractérisé en ce que** le substrat est sous forme de plaquettes et est constitué de mica, de SiO₂, d'oxyde d'aluminium, de verre, d'oxyde de fer micacé, de graphite oxydé, de graphite revêtu d'oxyde d'aluminium, de carbonate de plomb basique, de sulfate de baryum, d'oxyde de chrome, de BN, de MgO, de Si₃N₄, de métal, de pigments nacrants ou de pigments multi-couches nacrants, ou de sphères de SiO₂ revêtues ou non revêtues ou d'oxydes de fer en forme d'aiguilles.

14. Procédé selon la revendication 9, **caractérisé en ce que** l'oxyde, hydroxyde, oxyde mixte et/ou hydroxyde mixte est dopé par des ions métalliques, de l'oxyde de silicium, de l'oxyde d'aluminium, de l'oxyde de bore, du soufre, des ions phosphate et/ou des ions sulfate.

15. Procédé selon la revendication 14, **caractérisé en ce que** les métaux sont choisis parmi le silicium, le vanadium, le chrome, l'aluminium, le cérium, le néodyme, le praséodyme, le cobalt, le nickel et/ou le zinc.

16. Utilisation d'un pigment selon la revendication 1 dans des peintures, des revêtements pulvérulents, des revêtements de papier, des matières plastiques, des produits cosmétiques, des encres, des applications décoratives pour des aliments ou des médicaments et des éléments améliorant la sécurité.

17. Utilisation d'un pigment selon la revendication 1 comme matériau phosphorescent, fluorescent ou luminescent dans des applications de sécurité, optiques ou d'écran de projection.
